# EUROPEAN PATENT APPLICATION

(11) **EP 0 702 931 A1**
(43) Date of publication of application: **27.03.1996**
(21) Application number: 95306621.4
(22) Date of filing: 20.09.1995
(51) Int. Cl.: A61B 5/00

(54) **Noninvasive medical monitoring instrument**

(30) Priority: 20.09.1994 US 308900
(71) Applicant: OHMEDA INC., Liberty Corner, New Jersey 07938-0804 (US)
(72) Inventor: Dettling, Frederick Allen, Broomfield, Colorado 80020 (US); Ortiz, Michael Joseph, Lafayette, Colorado 80026-1213 (US); Pologe, Jonas Alexander, Boulder, Colorado 80303 (US)
(74) Representative: Gough, Peter

(57) **Abstract**

The medical monitoring instrument makes use of a monochromatic light source, such as a laser diode (21), to produce a high intensity light beam of narrow spectral bandwidth. The light source is located either in a monitor (2) or a cable (3) remote from a probe (1) attachment site on the patient and optical fiber (31) is used to transmit the light beam to the probe attachment site and optionally carry the return light signal back to the monitor (2). The operating temperature of the light source is precisely regulated by a control circuit (28) to ensure a light output of known wavelength.

## Description

This invention relates to medical monitoring instruments and, in particular, to apparatus that makes use of a monochromatic light source to noninvasively monitor blood analytes in a patient.

It is a problem in the field of medical monitoring instruments to manufacture a monitoring instrument that satisfies a number of diverse and sometimes contradictory requirements. It is important that the monitoring instrument be simple to use, in that it conforms to a variety of patients who differ in size and shape. The monitoring instrument must be securely affixable to the patient, such as on a patient's appendage, without requiring complex structures or elements that can irritate the patient. In addition, the probe must be either inexpensive so that it can be disposable after one use or able to be cleaned so that it can be used for many patient applications. If the probe is reusable, then the active elements contained therein that perform the sensing and measuring functions must be protected from the ambient environment and, likewise, it is a requirement that the patient be shielded from any potentially dangerous electrical signals or heat produced by the probe. The monitoring instrument must also reliably and accurately perform the required measurements.

In the specific field of photoplethysmography, the light beams that are generated by the monitoring instrument must be of sufficient intensity to illuminate the perfused tissue and also be of constant wavelength, since the light absorption of the monitored analyte varies as a function of wavelength. Light emitting diodes that produce light beams at red and infrared wavelengths are typically used in the probe for this purpose. The production of an intense beam of light must be balanced with the requirement that the probe does not operate at a significantly elevated temperature, which would cause irritation to the patient's skin. Furthermore, if the monitoring instrument is a hand held device, the power consumed by the light generation process must be minimized to conserve battery power, yet maintain sufficient light output to obtain adequate signal to noise ratios. A complicating factor is that the light emitting diodes are mounted in the probe module, and are juxtaposed to the patient's skin. The light emitting diodes are therefore subject to significant temperature fluctuations and the corresponding changes in wavelength output by the light emitting diodes, which causes a measurable source of error in the measurements that are taken by the monitor device.

Thus, there presently does not exist a monitoring instrument that can fully satisfy this plurality of diverse requirements in a manner that does not compromise the performance of the monitoring instrument.

According to the present invention, a medical monitoring apparatus for noninvasively monitoring blood analytes in a patient, comprises:
a probe module, affixable to an appendage of a patient, for illuminating perfused tissue in said appendage to measure light absorption by blood analytes contained in said perfused tissue, comprising:
light output means for applying a plurality of beams of light to said perfused tissue,
means for receiving light emanating from said illuminated perfused tissue;
control module, for producing light beams for use by said probe module, comprising:
a plurality of light emitting elements, each producing a substantially monochromatic beam of light; and
means interconnecting said control module and said probe module for coupling said plurality of beams of light produced by said control module to said light output means in said probe module.

The above-described problems are solved and a technical advance achieved in the field of medical monitoring instruments by the apparatus of the present invention which makes use of a monochromatic light source, such as a laser diode, to produce a high intensity light beam. The light source is located in a monitor, or alternatively in a cable connected to the monitor at a location remote from a probe attachment site on the patient. Optical fiber is used to transmit the light beam from the light source to the probe attachment site and optionally carry the return light signal back to the monitor.

In the preferred embodiment disclosed herein, the monitor and probe comprise an arterial blood monitoring instrument, such as a pulse oximeter instrument or total hemoglobin monitor. This apparatus makes use of light sources that generate substantially monochromatic light to noninvasively monitor blood analytes in the patient. The use of monochromatic light improves the accuracy of the measurements and simplifies the calibration process. The monochromatic light source is a laser diode, whose output is transmitted via optical fiber to the probe attachment site on the patient, where the optical fiber homogeneously transmits the light from a substantially point source into the perfused tissue. By placing the light source remote from the patient, there is no heat generated at the probe attachment site and EMI/EMC problems are reduced. The probe can be constructed to be more lightweight, conformable and inexpensive since the light generation and, optionally, light detection electronics are located remote from the probe attachment site. If the light detector is placed remote from the probe attachment site, the patient probe becomes a completely passive device, with no electrical connections, thereby simplifying the patient electrical isolation, probe encapsulation, and probe expense problems. Additional control electronics can be used in the light generation process since this function is implemented in totality as a part of the monitor itself and no part of this circuitry is part of the probe, which may be a disposable element. The additional control of the light generation process improves the performance of the medical monitoring instrument.

Thus, this monitor and probe architecture overcomes the problems inherent in existing probes and also is architected for ease of reliable and accurate manufacture. This monitor and probe architecture therefore represents a significant advance in the crowded technology of medical monitoring instruments.

An embodiment of the invention will now be described by way of example, reference being made to the Figures of the accompanying diagrammatic drawings in which:-
Figure 1 illustrates the architecture of the monitor and probe of the present invention, including a side cutaway view of the probe module and a block diagram of the control and light beam generation elements of the monitor;
Figures 2 and 3 illustrate one implementation of the probe module;
Figure 4 illustrates the multifiber optical coupler of the monitor;
Figure 5 illustrates a cross-section view of the multifiber optical coupler;
Figure 6 illustrates a waveform of the comparative light output of laser diodes and light emitting diodes; and
Figure 7 illustrates an alternative implementation of the probe module.

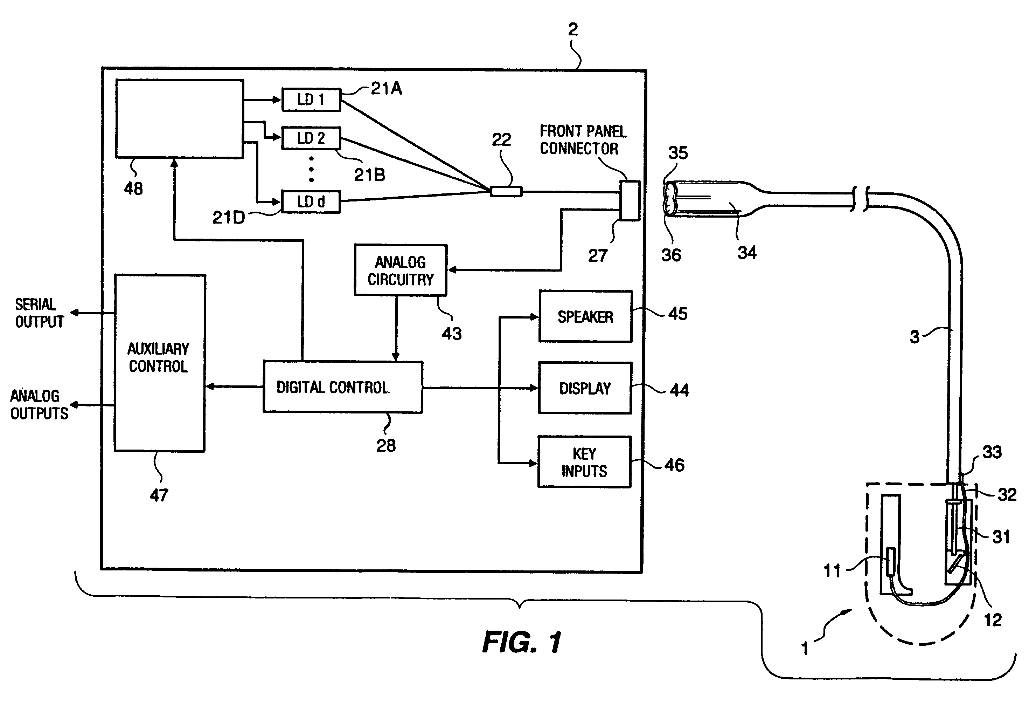

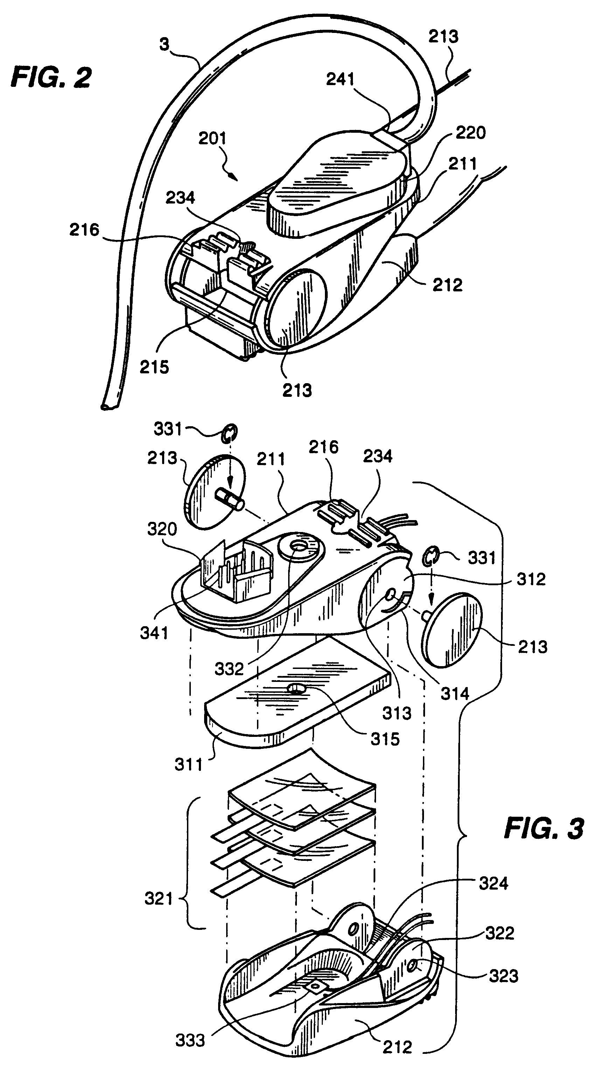

Any improvements, however incremental, in the design and manufacturability of a probe in the field of medical monitoring instruments result in significant cost savings. The apparatus of the present invention consists of two segments: an electronics (control) module, typically resident within the monitor, and a probe module that is affixed to a probe attachment site on the patient. Figure 1 illustrates the probe module in side cutaway view and a block diagram of the elements that comprise the control module. Figures 2, 3 and 7 illustrate alternative probe module implementations. The apparatus of the present invention represents an architecture in which the configuration of elements are cooperatively operative to solve the problems of existing medical monitoring instrument probes.

A pulse oximeter instrument is frequently used to monitor the condition of a patient in a hospital setting. The pulse oximeter instrument noninvasively measures the oxygen saturation of the arterial blood and produces a human readable display that indicates both the patient's heart rate and the oxygen saturation of the arterial blood. These readings are important to enable the medical staff to determine whether the patient's respiratory system is functioning properly, supplying sufficient oxygen to the blood.

A pulse oximeter instrument operates by use of a probe that transilluminates an appendage of the patient (such as a finger, earlobe, or the nasal septum) that is rich in arterial blood and measuring the amount of light that is absorbed by the pulsatile portion of the arterial blood thereby to determine oxygen saturation of the arterial blood. The pulse oximeter instrument makes use of a plurality of light-emitting devices, each of which transmits light at a predetermined wavelength, which wavelengths are selected such that at least one is highly absorbed by oxygenated hemoglobin in the arterial blood and at least one is highly absorbed by reduced hemoglobin in the arterial blood. The amount of absorption of the light beams generated by these light emitting devices that are located in the probe is a measure of the relative concentration of the oxygenated and reduced hemoglobin in the arterial blood. The absorption of the light that is being transmitted through the appendage of the patient includes a constant portion that is a result of skin, bone, steady-state (venous) blood flow and light loss due to various other factors. The pulsatile component of absorption is due to the pulsatile arterial blood flow and is a small fraction of the received signal and is used by the pulse oximeter instrument to perform its measurements.

The measurements are computed by periodically sampling the output of the light detector located in the probe to determine the incremental change in absorption of the various wavelengths of light transmitted through the appendage of the patient. These incremental changes in light absorption are then used to compute the oxygen saturation of the arterial blood as well as the patient's pulse rate. Since the pulsatile component of the signals received by the light detector represent only a small fraction of the incident light, it is important that the incident light be of significant magnitude to result in transmitted signals that have sufficient amplitude to provide accurate readings. In addition, the light-emitting devices and the light detector must be placed in intimate contact with the skin of the patient on opposite sides of the appendage (or on the same side of the appendage in reflectance probes) to obtain the most accurate readings. The probe design must therefore be such that it inherently accommodates variations in size and shape of the patient's appendage and also enables the medical staff to simply align the probe to obtain the maximum readings. These stringent requirements are difficult for existing probes to comply with and increase the manufacturing cost of the probes, which may be disposable elements.

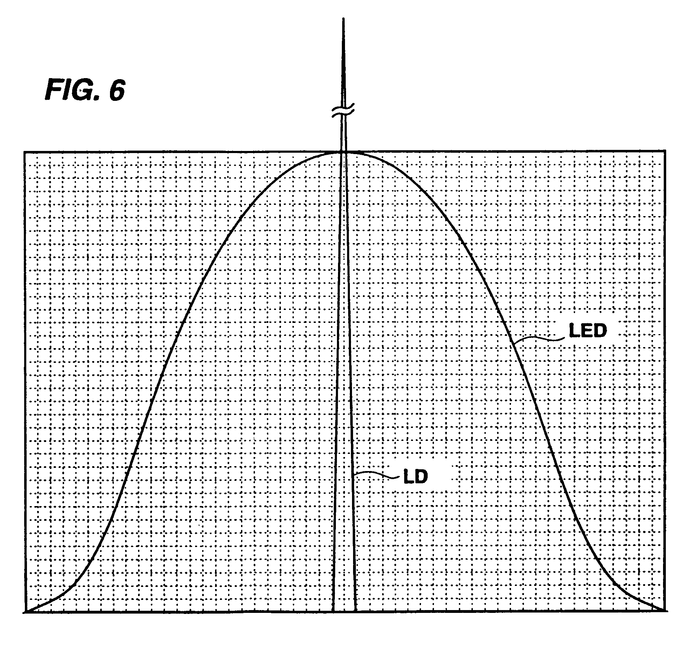

The light emitting devices used in the photoplethysmographic (pulse oximeter) instrument application disclosed herein are laser diodes, which produce an intense beam of light that is substantially monochromatic. The selection of laser diodes to generate the light beams that are used to illuminate the perfused tissue of the patient is a result of the preferable output characteristics of the laser diode as compared to the light emitting diode. Figure 6 illustrates the spectral output of these respective devices, with the laser diode producing a beam of light, whose spectral output (LD) is centered about a selected wavelength, such as 660nm, and having a bandwidth of as little as 1nm. The light emitting diode produces a beam of light, whose spectral output (LED) is centered about a selected wavelength, such as 660nm, and having a bandwidth of as wide as 60nm. Thus, the laser diode is able to generate a great deal of energy over a narrow spectral range when compared to a light emitting diode.

In addition to the extent of the bandwidth, the variation in bandwidth among light emitting diodes represents a significant problem. The tail end of the spectral emissions of the light emitting diode can have a measurable effect on the received signal from the perfused tissue. The magnitude and extent of the tail portion of the spectral emissions is more difficult to measure and account for than the signal output at maximum intensity. A complicating factor is that the light emitting diodes are mounted in the probe module, and are juxtaposed to the patient's skin. The light emitting diodes are therefore subject to significant temperature fluctuations and the corresponding changes in wavelength output by the light emitting diodes, which cause a measurable source of error. Finally, another source of error is the "venous prefiltering", wherein the spectral output of the light emitting diodes is unevenly and unpredictably attenuated by the presence of the venous and non-pulsatile arterial components of the blood. This attenuation of the light is a function of the oxygen saturation of the blood and wavelength, varies from subject to subject, and also is temporal in nature, varying within a given patient. The arterial blood flow is highly variable in the extremities of a patient, where the pulse oximetry readings are taken. The difference in oxygen saturation between arterial and venous components of the blood can be from as little as less than one percent to greater than twenty-five percent. Thus, the greater the spectral bandwidth, the greater this source of error. The light emitting diode and the method of placing it on the patient's appendage to perform readings have inherent sources of error that cannot be accurately quantified or compensated.

The apparatus of the present invention consists of two primary segments: a control module 2, typically resident within the monitor, and a probe module 1 that is affixed to a probe attachment site on the patient. These two modules 1,2 are interconnected by a cable 3 which can be affixedly attached to and an integral part of probe module 1 or can be an element that is connectorized at both ends thereof to enable cable 3 to be disconnected from both probe module 1 and control module 2. In the embodiment disclosed herein, cable 3 is shown as part of probe module 1.

The control module 2 primarily consists of the light generation apparatus but also includes circuitry to receive the return signals produced by the reception of light that passes through the illuminated appendage as well as user interface circuitry. A plurality of laser diodes 21A-21D are noted in Figure 1, labeled as "LD1, ... LDd". The number of laser diodes 21A-21D is equal to the number of blood analytes that are to be measured by the instrument. For standard pulse oximetry, the number of laser diodes can be as little as two, one whose light output is at a wavelength of approximately 660nm and a second whose light output is at a wavelength of approximately 940nm. In the system disclosed herein, four laser diodes 21A-21D are disclosed.

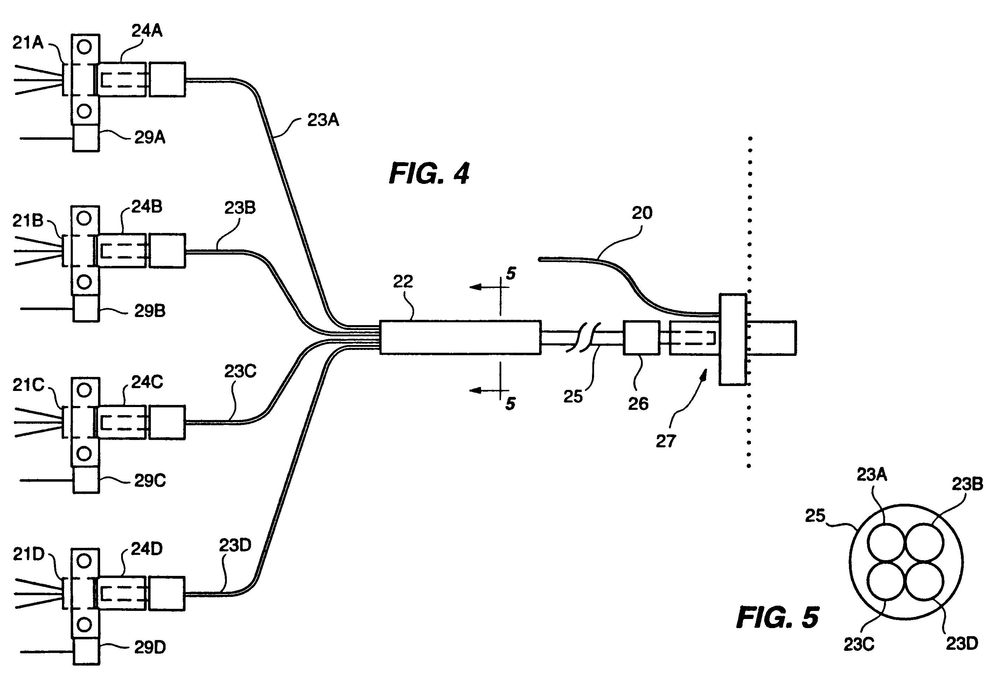

The wavelength of the light beams output by the laser diodes 21A-21D are affected by the operating temperature of the laser diodes 21A-21D. Since the light emitting devices, the laser diodes 21A-21D, are located in the monitor and not in the probe module 1, additional control circuitry can conveniently be added to regulate precisely the temperature of the light emitting devices 21A-21D and/or compensate for variations in their temperature. In prior systems where the light emitting diodes are located in the probe module 1, these devices must be of minimalistic design since they may be disposable and to also reduce the bulk of the probe. As shown in additional detail in Figure 4, the control module 2 includes at least one and possibly four temperature sensing elements, such as thermistors 29A-29D, each of which are attached to a optical device mount 24A-24D to monitor the operating temperature of the corresponding laser diode 21A-21D. Alternatively, thermocouple, silicon sensor, or other temperature-electric transducers can be used. As the temperature in the laser diode cavity changes, the temperature of the heat sink also changes. The thermistor's resistance varies in a mathematically definable manner, typically monotonically, with temperature. The measured operating temperature, in the form of an analog voltage output by the thermistor, is then forwarded to an analog-to-digital converter (A/D) circuit (not shown) that is part of digital control circuit 28. The digital output from the A/D converter is used by the digital control circuit 28 using a non-linear equation to produce an indication of the measured temperature of the laser diodes 21A-21D. The spectral content of the light beam emitted from each laser diode 21A-21D is a function of the operating temperature of the laser diode 21A-21D. The probe system is designed and calibrated for specific known spectral content for each laser diode 21A-21D and also dynamically compensates for temperature variations of this spectral content. The digital control circuit 28 adjusts the characteristic factors for the laser diodes 21A-21D to compensate for any variation in wavelength output, since the output wavelength is a known function of the operating temperature. In the pulse oximetry example used herein, the precise determination of operating wavelength translates to a precise locus on the pulse oximetry characteristic curve, thereby improving the accuracy of the measured oxygen saturation. Thus, the blood analytes can be more accurately measured since spectral variation of the laser diodes 21A-21D are automatically and dynamically compensated. The control module 2 includes analog circuitry 43 that functions to receive and condition the electrical signals produced by the light detector 11 that is contained within the probe module 1, as is well known in the art. These received signals are digitized and transmitted to digital control 28 for processing as is well known in the field to produce the required measurements. The digital control 28 outputs the computed measurements to display 44 for production of a human-readable output. A speaker 45 is typically included to enable digital control 28 to produce audible alarms to alert the user to error or danger conditions. Key inputs 46 are also typically provided to enable the user to input control information to regulate the operation of the instrument. An auxiliary control circuit 47 receives output signals from digital control circuit 28 for transmission in serial and/or analog form to other elements in the medical monitoring instrument.

The light beams generated by the laser diodes 21A-21D are transmitted to the probe module 1 via an optical fiber 31 that is part of cable 3. The multifiber optical coupler 22 shown in Figure 1 performs the task of coupling the optical output of all the laser diodes 21A-21D on to the optical fiber 31. The multifiber optical coupler 22 is illustrated in additional detail in Figure 4 and in cross section view in Figure 5. The plurality of optical fibers 23A-23D are joined together in a manner to couple the light beams that are produced by the plurality of laser diodes 21A-21D into a single optical fiber 31 for transmission to the probe module 1.

Each of the laser diodes 21A-21D is mounted in an optical device mount 24A-24D which functions to interconnect the laser diode 21A-21D with an optical fiber 23A-23D. The optical device mount 24A-24D is constructed to accept an optical fiber 23A-23D and precisely align the end face of the optical fiber 23A-23D with the light output of the laser diode 21A-21D to thereby couple the light beam produced by the laser diode 21A-21D into the optical fiber 23A-23D. The plurality of optical fibers 23A-23D are terminated at the distal end thereof in a multifiber optical coupler 22. The multifiber optical coupler 22 functions to interconnect the plurality of optical fibers 23A-23D with a single intermediate optical fiber 25. Figure 5 illustrates a cross-section view (taken across section 5-5 of Figure 4) of the multifiber optical coupler 22, wherein the four optical fibers 23A-23D are grouped together and their end faces are optionally butted to the end face of a larger diameter intermediate optical fiber 25. The configuration shown in Figure 5 is such that the single intermediate optical fiber 25 has a diameter greater than the cross section of all four individual optical fibers 23A-23D grouped together. The multifiber optical coupler 22 ensures that little if any light is lost in the transition from multiple optical fibers 23A-23D to the single intermediate optical fiber 25. The single intermediate optical fiber 25 is itself terminated at the end distal from the multifiber optical coupler 22 in a connector 26, which interconnects the intermediate optical fiber 25 with a hybrid connector 27, located on the front panel of the pulse oximeter instrument. The multifiber optical coupler 22 may be eliminated so that the four optical fibers 23A-23D are grouped together in a connector 26 which interconnects the four optical fibers 23A-23D directly with a hybrid connector 27 located on the front panel of the pulse oximeter instrument.

The pulse oximeter instrument is a connectorized system, with the cable 3 that interconnects the probe module 1 with the control module 2 being detachable from the connector 27 located on the front panel of the pulse oximeter instrument. The cable 3 is a hybrid cable, consisting of an optical fiber 31 and electrical conductor 32 combination contained within a single sheath 33.

The cable 3 therefore includes optical fiber 31 which carries the plurality of light beams generated by the laser diodes 21A-21D as well as electrical conductors 32 which interconnect the light detector 11 of probe module 1 with the processor circuit 28 in control module 2. Connector 27 can have two adjacent connector elements 35, 36 with a first 35 being the optical connector and a second 36 being the electrical connector.

The probe module 1 consists of the light detector 11 and optical apparatus that applies the light beams carried by the optical fiber 31 to the patient's appendage. This optical apparatus consists of a reflective surface such as a mirror 12 mounted at an angle with respect to the end face of optical fiber 31 to reflect the incoming light beam that emanates from the end of optical fiber 31 into living tissue, such as the patient's skin, so that the reflected light beam is orthogonal to the skin surface. This configuration allows the optical fiber 31 to enter the probe module 1 parallel to the long axis of the patient's finger, while applying the incident light perpendicular to the patient's skin. The mirror 12 is mounted along with optical fiber 31 in an optical mount 13 which is typically affixed to cable 3 to ensure proper positioning of the optical elements within the probe module 1. A lens is optionally provided to direct precisely the incident light directly on to the patient's skin. The end face of the optical fiber 31 is positioned by mount 13 adjacent to the mirror 12 which reflects the light beam that emanates from the end of optical fiber 31 at a 45° angle to the patient's finger. A common aluminum front surface mirror typically allows approximately 70% of the light at wavelengths of 635nm and 1310nm to be reflected. The use of a gold front surface mirror improves the reflectivity to approximately 97%. By making the mount 13 and mirror 12 an integral part of the cable 3, the probe module 1 becomes an inexpensive element, since the cost of manufacture is reduced by this simple yet accurate cable and mirror placement.

Figures 2 and 3 illustrate perspective and exploded views of one implementation of a probe module 1. This configuration is a "clip-tip" or "spring clip" probe that makes use of a spring loaded pair of opposing arms 211, 212 to place precisely the patient's finger in the proper position with respect to the optical port 332 and light detector 333 elements and to place these elements in contact with the patient's skin at the probe attachment site. The spring clip probe module 1 consists of a first arm 211, which is equipped with a conformable pad 311 attached to the interior surface of first arm 211 and having an aperture 315 to permit light to pass from optical port 332 to the patient's finger that is placed in the spring clip probe. Also included in the first arm 211 is a connector 220 for interconnecting a cable 3 to the light detector 333 included in the spring clip probe 1 and positioning the optical fiber 31 and mirror 12 over the optical port 332. The second arm 212 of spring clip probe 1 includes light detector 333 and an adhesively coated material 321 that serves to affix the spring clip probe 1 to the patient's finger 213. The first arm 211 and the arm 212 are similar in configuration and, when fitted together, function to press the optical port 332 and light detector 333 against a patient's finger 213 that is inserted between the first 211 and second 212 arms. The first 211 and second 212 arms are hingeably attached to each other and include a spring member 314 integral with the first arm 211 that functions to bias the first 211 and second 212 arms together in a closed position as illustrated in Figure 2. First 211 and second 212 arms include mating pieces that, when assembled with a pivot pin 214, function as a hinge. In particular, the first arm 211 includes two areas 312, each of which has an aperture 313 formed therein to correspond to a mating aperture 323 in areas 322 on the second arm 212. The first 211 and second 212 arms are aligned and interconnected by the insertion of pivot pins 214 in the respective apertures 313, 323 when oriented opposite each other such that the tissue contacting surfaces of the interior surfaces of first and second arms 211, 212 face each other. The integral spring 314 that is part of first arm 211 exerts a force against the second arm 212 to force the ends of the first and second housing arms 211, 212 apart, which cause the first and second housing sections 211, 212 to rotate with respect to each other around the pivot pins 313, thereby forcing the other ends of the housing together.

The interior surface of the first 211 and second 212 arms include a curved portion to substantially parallel the contours of a typical finger 213 to which the spring clip probe module 1 is connected. The conformable pad 311, and adhesively coated material 321 function to compensate for topological differences between the patient's finger 213 and the curvature of the inside of the two sections 211, 212 of the spring clip probe 1. Thus, when the two arms 211, 212 of the spring clip probe module 1 are closed about a patient's finger 213, the conformable pad 311 and adhesively coated material 321 form a surface that substantially maps to the contours of the patient's finger 213. The use of the conformable pad 311 and adhesively coated material 321 and the spring mechanism 314 of the spring clip probe module 1 ensures that the optical port 332 and the light detector 333 are placed in close and firm contact with the skin of the patient's finger 213. The close contact of the optical port 332 and the light detectors 333 with the patient's finger 213 is critically important since any ambient light that is received by the light detector 333 interferes with the measurement of the particular characteristics of the arterial blood that are performed by the monitoring equipment.

The interior surface of the second arm 212 is also shaped to match the contours of the bottom of a finger 213 such that the interior end 324 of this recess functions as a finger stop which is designed to position the patient's finger 213 inside of the housing section 212 at a predetermined location. The height of the finger stop 324 is designed to permit a fingernail, especially a long fingernail, to pass over the top, but also to prevent the fleshy fingertip from extending beyond a selected point between the two arms 211, 212 of the spring clip probe module 1. The light detector 333 is mounted in the bottom of the contoured area of second arm 212 in a predetermined location.

In order to prevent the movement of the patient's finger 213 within the spring clip probe module 1, the adhesively coated material 321 located in the second arm 212 is implemented by means of at least one and preferably a plurality (shown in Figure 3) of layers of clear conformable material 321 that are adhesively coated on both sides thereof. The conformable pad 311 adhesively affixed to the first arm 211 can be manufactured of an opaque material having an aperture (hole) 315 cut therein to allow the optical port 332 affixed to the interior surface of the first arm 211 of the spring clip probe 1 module to transmit light through the hole 315 in the conformable pad 311 to shine on the patient's finger 213 at a predetermined location, such as near the cuticle of the finger.

The construction of the first arm 211 and second arm 212 of the spring clip probe module 1 is such that, when closed on the patient's finger 213, the first 211 and second 212 arms have surfaces conforming to the patient's finger 213 and close akin to a clam shell. The hingeably connected ends of the first 211 and second 212 arms of the spring clip probe module 1 are cut such that in a closed position an aperture 215 is provided between the first 211 and second 212 arms. This aperture 215 is closed when the first 211 and second 212 arms are opened to the full extent, the range of travel being determined by the size of the aperture 215. Therefore, when placed in a fully opened position the pivoting end of the first 211 and second 212 arms encounter each other, restricting the range of motion of the first 211 and second 212 arms.

The light detector 333 is of conventional design typically found in pulse oximeter probes. The plurality of conductors are connected to the light detector 333 and these conductors are terminated in a connector 220 located on the top surface of the first arm 211 of the spring clip probe module 1. This connector 220 includes a plurality of pins 341 arranged in a predetermined pattern therein, which connector 220 is located at the patient end of the spring clip probe 1 in order to provide the user with sufficient space to grasp the spring clip probe module 1 for application to the patient's finger 213.

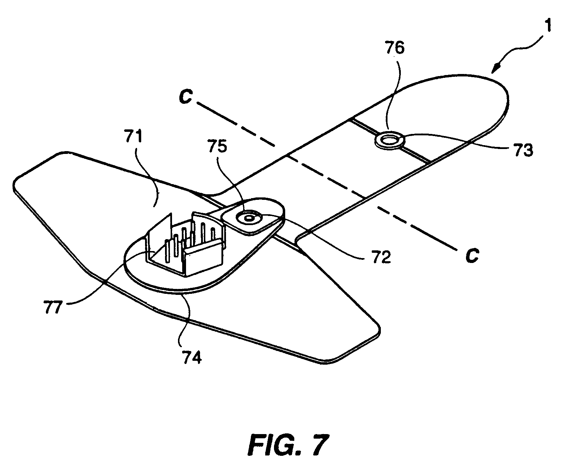

The probe module 1 as shown in the embodiment of Figure 7 consists of a connector 77 attached by cable 3 to the probe module 1 which includes a sealed housing 71 that provides a moisture barrier and an electrical barrier to prevent the exchange of contaminants or electrical signals between the light source 72, light detector 73 and the ambient environment that is extant around the probe module 1. The seal 74 on exterior housing 71 around connector 77 serves to seal the probe module 1. The sealing process can be a heat sealing process although other sealing processes, such as adhesive, ultrasonic bonding, etc. can be used dependent on the nature of the materials used to implement the layers of probe module 1. The sealed probe module 1 protects light source 72 and light detector 73 from fluids, such as the cleaning and disinfectant fluids used on the probe module 1 prior to its reuse.

The two circles 75, 76 illustrated on one side of the probe module 1 represent the optical ports through which the light beams produced by the laser diodes 21A-21D and transmitted via optical fiber 31 to the probe module 1 are output to the patient's appendage and the light received from the passage of these light beams through the patient's appendage is received by the light detector 11, respectively. The bottom surface of the exterior housing 71 is an adhesive element that is used to adhesively affix the probe module 1 to the patient's appendage. In use, the ends of the probe module 1 are folded together to form a U-shaped structure, folded around the center line C illustrated in Figure 7. This U-shaped configuration enables the probe center section to conform to the shape of the appendage of the patient and the integral adhesive secures the probe module 1 to the patient's appendage. This unitary structure is therefore simple in architecture, self-contained in that it may not require the use of adhesive strips that must be wrapped around the patient's appendage, and not of a bulky construction that is difficult to apply and secure to the patient.

The apparatus of the present invention makes use of a monochromatic light source, such as a laser diode, to produce a high intensity light beam. The light source is typically located in the monitor remote from the probe attachment site on the patient and optical fiber is used to transmit the light beam to the probe attachment site. The use of monochromatic light ensures that the transmitted wavelength is a known, improves the accuracy of the measurements and simplifies the calibration process. By placing the light source remote from the patient, there is no heat generated at the probe attachment site and EMI/EMC problems are reduced.

## Claims

1. A medical monitoring apparatus for noninvasively monitoring blood analytes in a patient, comprising:
a probe module 1, affixable to an appendage of a patient, for illuminating perfused tissue in said appendage to measure light absorption by blood analytes contained in said perfused tissue, comprising:
light output means 12,13 for applying a plurality of beams of light to said perfused tissue,
means 11 for receiving light emanating from said illuminated perfused tissue;
control module 2, for producing light beams for use by said probe module 1, comprising:
a plurality of light emitting elements 21A to 21D, each producing a substantially monochromatic beam of light; and
means 3 interconnecting said control module 2 and said probe module 1 for coupling said plurality of beams of light produced by said control module 2 to said light output means in said probe module 1.

2. An apparatus as claimed in claim 1 wherein said light output means 12, 13 comprises means connected to said coupling means 3 for directing said plurality of beams of light received from said coupling means 3 to said perfused tissue.

3. An apparatus as claimed in claim 2 wherein said coupling means 3 includes an optical fiber 31 for carrying said plurality of light beams; said light output means further comprising means for positioning said directing means at an end of said optical fiber 31, wherein said directing means 12 is in a path of said light beams as they emanate from said end of said optical fiber.

4. An apparatus as claimed in claim 3 wherein said directing means is a mirror 12 for reflecting said light beams received from said end of said optical fiber 31 to enter said perfused tissue at a predetermined angle.

5. An apparatus as claimed in any one of claims 1 to 4 wherein said plurality of light emitting elements comprises a plurality of laser diodes 21A, 21B, 21C, 21D for generating a plurality of beams of light, each of which is at a predetermined wavelength, wherein each of said predetermined wavelengths differ from each other.

6. An apparatus as claimed in any of claims 1 to 5 wherein said coupling means 3 comprises a single optical fiber 31, said control module 1 further comprising means 22 for optically coupling said plurality of beams of light into said single optical fiber 31 contained in said cable means 3.

7. An apparatus as claimed in claim 5 wherein said light receiving means 11 generates an electrical signal indicative of a magnitude of said received light that emanated from said illuminated appendage, said control module 2 further comprising:
means for measuring operating temperature of said plurality of laser diodes 21A to 21D; and means, responsive to said measured operating temperature, for determining said predetermined wavelength of said plurality of beams of light.

8. An apparatus as claimed in claim 7 wherein said control module 2 further comprises:
means for computing characteristics of said blood analytes as a function of said magnitude of received light; and
means for adjusting operation of said computing means as a function of said determined wavelength of said plurality of beams of light.

9. An apparatus as claimed in claim 1 wherein said probe module comprises:
housing means 71 having at least two outer layers sealed together to form an integral structure for enclosing said light output means 72 and said light receiving means 73.

10. An apparatus as claimed in claim 1 wherein said probe module 1 comprises:
a first housing section 211;
a second housing section 212 hingeably attached to said first housing section 211 to form a clamshell-type housing, said first and said second sections being rotatable between an open and a closed position and having an aperture in one end thereof when in said closed position to receive said appendage of a patient, with said first and said second housing sections both having an interior surface facing each other and an exterior surface
